# EUROPEAN PATENT SPECIFICATION

(11) **EP 3 449 249 B1**
(45) Date of publication and mention of the grant of the patent: **25.12.2019**
(21) Application number: 17727406.5
(22) Date of filing: 01.05.2017
(51) Int. Cl.: G01N 33/04

(54) **FRESH GRAZED GRASS INDICATOR**
INDIKATOR FÜR FRISCHES ABGEGRASTES GRAS
INDICATEUR D'HERBE BROUTÉE FRAÎCHE

(30) Priority: 29.04.2016 NL 2016707
(43) Date of publication of application: 06.03.2019
(73) Proprietor: Qlip B.V., 7202 CM Zutphen (NL)
(72) Inventor: RADEMAKER, Johannes Laurentius Wouterus, 7202 CM Zutphen (NL); DE PEINDER, Peter, 7202 CM Zutphen (NL); SHETTY, Nisha, 7202 CM Zutphen (NL)
(74) Representative: V.O.
(86) International application number: PCT/NL2017/050274
(87) International publication number: WO 2017/188819

(56) References cited:
- CAPUANO EDOARDO ET AL: "Verification of fresh grass feeding, pasture grazing and organic farming by cows farm milk fatty acid profile", FOOD CHEMISTRY, vol. 164, 15 May 2014 (2014-05-15), pages 234-241, XP028861055, ISSN: 0308-8146, DOI: 10.1016/J.FOODCHEM.2014.05.011
- COUVREUR S ET AL: "The Linear Relationship Between the Proportion of Fresh Grass in the Cow Diet, Milk Fatty Acid Composition, and Butter Properties", JOURNAL OF DAIRY SCIENCE, AMERICAN DAIRY SCIENCE ASSOCIATION, US, vol. 89, no. 6, 1 June 2006 (2006-06-01), pages 1956-1969, XP026957022, ISSN: 0022-0302 [retrieved on 2006-06-01]
- ELGERSMA A ET AL: "Quick changes in milk fat composition from cows after transition from fresh grass to a silage diet", ANIMAL FEED SCIENCE AND TECHNOLOGY, ELSEVIER, AMSTERDAM, NL, vol. 117, no. 1-2, 10 November 2004 (2004-11-10), pages 13-27, XP004593432, ISSN: 0377-8401, DOI: 10.1016/J.ANIFEEDSCI.2004.08.003
- A.L LOCK ET AL: "Seasonal variation in milk conjugated linoleic acid and [Delta]9-desaturase activity in dairy cows", LIVESTOCK PRODUCTION SCIENCE., vol. 79, no. 1, 1 January 2003 (2003-01-01), pages 47-59, XP055333875, NL ISSN: 0301-6226, DOI: 10.1016/S0301-6226(02)00118-5

## Description

The invention is in the field of analytics, in particular analytics in the dairy industry. The invention is directed to a method for determining feed intake of a dairy cow population (herd), in particular for determining the fresh grazed grass intake of dairy cows.

The composition of milk produced by dairy cows is known to be influenced by the feed that is ingested by the dairy cows. Some feed, for instance fresh grazed grass, is considered beneficial for the quality of the milk. Additionally, dairy cows that are allowed to graze fresh grass are perceived as more natural, as healthy and to receive better animal care compared with dairy cows that are fed freshly mown grass, grass or corn silage grains, and the like. In the Netherlands, for instance, milk produced by dairy cows which have been kept grazing in a pasture for at least six hours per day for a period of at least 120 days or 720 hour in total may be labelled as *"weidemelk",* a Dutch term that can be translated as "meadow milk". The commercial value of meadow milk is typically higher than non-pasture-based milk and the origin of the milk and the stay of the dairy cows in a pasture is therefore monitored and documented in order to prevent mislabeling.

Conventional methods to monitor the stay of cows in a pasture and whether the cows can graze fresh grass includes visual inspection by periodically visiting the farm. Alternatively sensor based systems are available to monitor behavior and position (presence in the meadow or not) of cows. These methods however are laborious, costly and/or prone to false conclusions as the observations during the inspection may not be representative for a longer period or equipment may be sensitive to technical errors. Moreover these measures are not based on the produced milk, but on farm management and animal behavior. It is therefore preferred that the fresh grazed grass ingestion can be determined by an analytical methodology of the produced milk.

A number of attempts have been made to determine fresh grazed grass ingestion by analyzing a sample of milk. For instance, in Capuano et al. Food Chemistry, 164, 2014, pages 234-241, an attempt is described to distinguish milk from cows at pasture from milk from stabled cows without fresh grass in the diet or from milk from stabled cows fed fresh grass indoors. It was found that unequivocal discrimination was not possible either in summer or in winter.

The present inventors have surprisingly found that the ingestion of fresh grazed grass can be determined if the milk of the dairy cows is analyzed that is collected over a longer grazing period and compared to the milk that is collected over a longer reference period from the same cows. It was found that the absolute and/or relative amount of several components in and/or color of milk can vary due to difference in farming methods, ingestion of other feed than fresh grazed grass, type of grass, seasons, weather conditions, countries, physical condition of the cow(s) and other influencing factors, besides the amount of fresh grazed grass ingestion. By merely analyzing one or a few samples, the variation in biomarkers, *i.e.* milk characteristics and/or components, *e.g*. color or fatty acids (FAs) caused by these influencing factors generally result in high levels of inaccuracy of the method. However, by comparing multiple milk samples from the dairy cows that are collected over a longer grazing period to multiple milk samples that are collected over a longer reference period from the same cows, the variation in milk biomarkers caused by the amount of fresh grazed grass intake is apparent. Thus the present invention can distinguish between a high likelihood of substantial fresh grazed grass intake and a low likelihood of such intake.

The present invention is therefore directed to a method for determining fresh grazed grass intake over a grazing time period for a cow population, said method comprising the steps of:
a) providing a number of milk samples that are collected from the cow population over said grazing time period and over a reference time period without fresh grass grazing;
b) analyzing said milk samples and determine the amount of one or more biomarkers present in each of the milk samples;
c) comparing the amount of the biomarkers present in the milk during said grazing time period to the amount of the biomarkers present in the milk during the reference time period to obtain an indicator of fresh grazed grass ingestion for the cow population over the grazing time period.

The indicator can be expressed conveniently as a number. The value of such a number can be chosen freely, depending on how the different factors making up the indicator are weighed and combined. The indicator may for instance be expressed as the likelihood-ratio as used in the likelihood-ratio test. The likelihood-ratio test is a well-known statistical test used to compare the goodness of fit of two models, one of which (the null model) is a special case of the other (the alternative model). The test is based on the likelihood ratio, which expresses how many times more likely the data are under one model than the other. This likelihood-ratio can then for instance be used to compute a p-value, or compared to a critical value to decide whether to reject the null model in favor of the alternative model.

The method has been found particularly suitable for determining fresh grazed grass ingestion on farm level. As such, the cow population is typically located on a single farm. On a farm, all the milk collected from the dairy cow is typically combined in a single reservoir before it is transported to a milk distribution center. It is preferred to provide milk samples from this herd reservoir and herewith determine the fresh grazed grass ingestion of the lactating cows for said farm as a whole. Alternatively, the cow population can also be considered to be a single cow by providing and analyzing the milk samples in accordance with the present invention collected from a single cow.

When the indicator (such as the likelihood of fresh grazed grass intake) is known, this can be used for instance to decide whether additional visits to a specific farm are required, in particular when the likelihood is too low.

When the cow population consists of more than one cow, the cow population may slightly vary over time, *e.g*. a number of cows may join and/or leave the population. However, by providing and analyzing a sufficient number of samples over time and/or by limiting this variation, the desired accuracy can be obtained. Variations in milk composition and/or color correlated to a varying cow population and/or by the influencing factors as described herein above are thus cancelled out by regarding a sufficient number of milk samples. This applies to any cow population; whether or not it is located on a single farm. In general, it is preferred that the number of milk samples analyzed for each time period is at least 10, preferably at least 25. The number of samples that are collected is not necessarily the same for each time period. For instance, more samples may be collected for the grazing time period (*e.g*. 15) than for the reference time period (*e.g*. 12).

The grazing time period is typically a summer period and the reference time period is normally a winter period. During the summer period, the cow population is generally spending more time in the pasture than during the winter period. In fact, on a typical farm, the cow population is kept in a barn or stable during the whole winter period so that it cannot graze fresh grass. This winter period may thus serve as a reference time period. A cow population that may graze fresh grass is generally enabled to do so during the summer period when grass grows well. For the northern hemisphere, it is preferred that the grazing time period spans at least 30 days selected from days within the period of April to September and wherein the reference time period spans at least 30 days selected from days within the period of December to February. For the southern hemisphere, this may be vice versa.

Depending on regulations, milk of farms and other cow populations are generally analyzed every few days (*e.g*. each three days) to determine the composition, quality and the corresponding value of the milk. For instance, for a typical farm in the Netherlands, every three days the milk of a farm is sampled and analyzed. The milk on a farm is generally collected in a single reservoir (farm tank). It is preferred that the samples that are collected and analyzed to determine the value of the milk are also used to determine the indicator of the present invention. This prevents the undesirable requirement of additional infrastructure. By providing a milk sample every three days from a cow population, sufficient samples (*e.g*. 10) can be obtained within a period of 30 days. A number of less than 10 samples may also be used, provided that the desired accuracy is still obtained. By providing milk samples every three days for a period of three months (*e.g*. June to August for the grazing time period and December to February for the reference period), a total number of 30 samples can be obtained for each time period.

Using the present method, a cow population grazing fresh grass can be distinguished from a cow population that does not ingest fresh grazed grass but only ingests mown grass or silage. This can be achieved by periodically regarding certain milk characteristics and/or components, *e.g*. color or fatty acids (FAs), in the milk as biomarkers.

The content of FAs in grass, and in particular polyunsaturated FAs (*i.e.* PUFAs such as linoleic or linolenic acid), is determined by the genetics of the grass and by environmental influencing factors such as light, mowing frequency, age, season, use of fertilizer and the like. Additionally, during mowing of grass and conservation of mown grass, unsaturated FAs may degenerate and be lost. A cow may also give her preference to a certain part of unmown grass (*e.g.* the tender parts of grass such as the leaves) and based on this preference, the cow may select which part of the grass she ingests. In contrast, selecting certain parts from mown (mingled) grass is less possible. The (nutritional) composition of the parts which a cow prefers to graze and ingest generally differs in FA composition from the other parts of the grass. These differences in FA content may be exploited for the present invention by using one or more FAs in the milk samples as biomarkers.

A number of analytical methods exist to quantitatively determine the components such as fatty acids and proteins present in milk. For instance, in Capuano et al. Food Chemistry, 164, 2014, pages 234-241 the use of gas chromatography is described. Alternatively, Fourier transform infrared (FT-IR) methods can be used. FT-IR is preferred since it enables high throughput and is a well-established method to analyze milk samples routinely, time-efficient and cost-effective.

A number of analytical models are known to quantitatively establish the FA content in milk by FT-IR. For instance, Rutten et al. in Journal of dairy science, 92(12), 6202-6209, US4247773 and US2009/019734, which are incorporated herein in their entirety, each describe models for quantitative determination of FAs in milk by using FT-IR. These models may also be used in the present invention. A suitable instrument to be used for the present invention is a FT-IR apparatus commercially available from Foss (Hillerød, Denmark) such as the MilkoScan FT 6000 equipment. This and similar instruments (e.g. from Delta instruments, Drachten, Netherlands; Bentley Instruments Inc., Chaska, Minnesota, USA; Bruker Ettingen, Germany) are generally provided including analytical models to determine total FA content in milk.

The present inventors realized that a suitable biomarker is a component in milk of which the amount is different in the grazing time period compared to the amount in the reference time period. The amount of biomarker may be higher or may be lower in the grazing period compared to the reference period. Preferably, the type of biomarker can be used for any cow population and thus not only for a specific cow population. A number of fatty acids were found that meet this requirement.

In step b), the amount of one or more biomarkers may be determined. Preferably, more than one type of biomarker is determined and the amount of each biomarker is different in the grazing time period compared to the amount of the respective biomarker in the reference time period. This further improves the accuracy of the method.

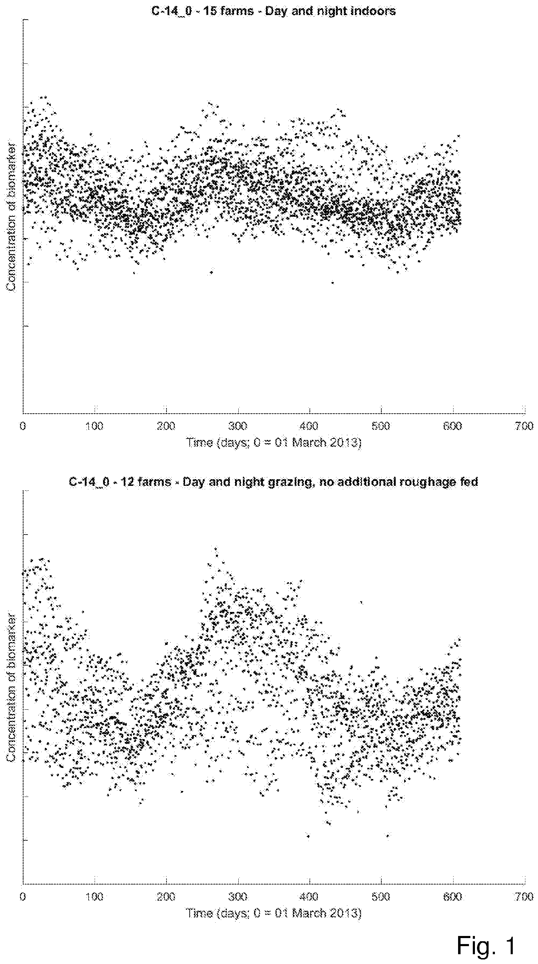

In Figure 1, a plot is provided showing the amount of a fatty acid in milk, plotted against the time.

Preferably, the biomarker is selected from milk components comprising in particular fatty acids selected from the group consisting of C14:0 (myristic acid), C16:0 (palmitic acid), total saturated FAs, C18:1 trans 11 (vaccenic acid), C18:2 cis 9, trans 11 (*viz.* CLA, *i.e.* conjugated linoleic acid; more specifically: rumenic acid) and C18:3 cis 9,12,15 (*viz.* ALA, *i.e.* α-linolenic). It may be preferred that a combination of these biomarkers is used. For instance, the combination of the biomarkers C16:0 and C18:1 trans 11 has shown particularly good results.

The notation used herein to describe the structure of the FAs is based on C(*number of carbon atoms*):(*number of double bonds*) (*geometrical configuration of double bond, i.e. cis or trans*)(*location of double bonds*). C18:1 trans 11 thus notates a C₁₈ FA with one *trans* double bond located between carbon atoms 11 and 12 counting from the carboxylic acid end.

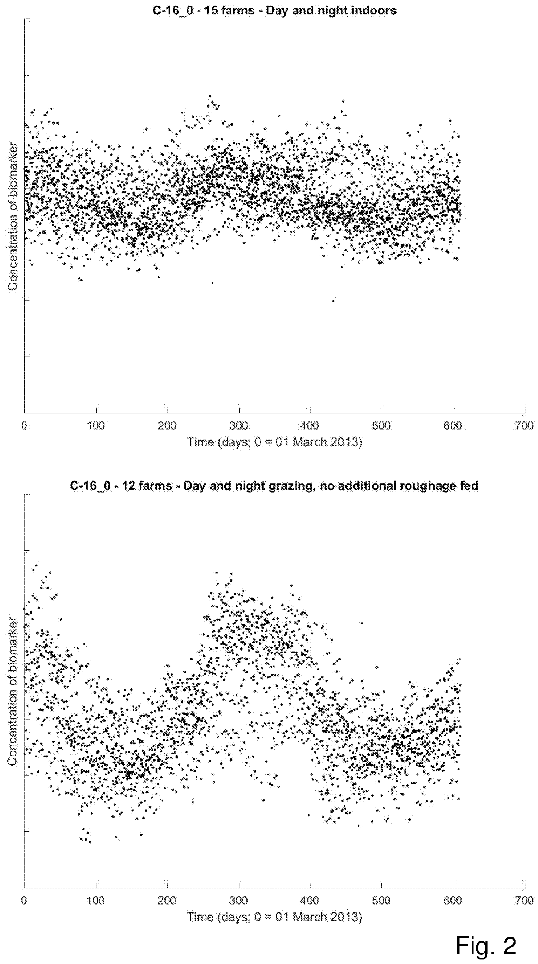

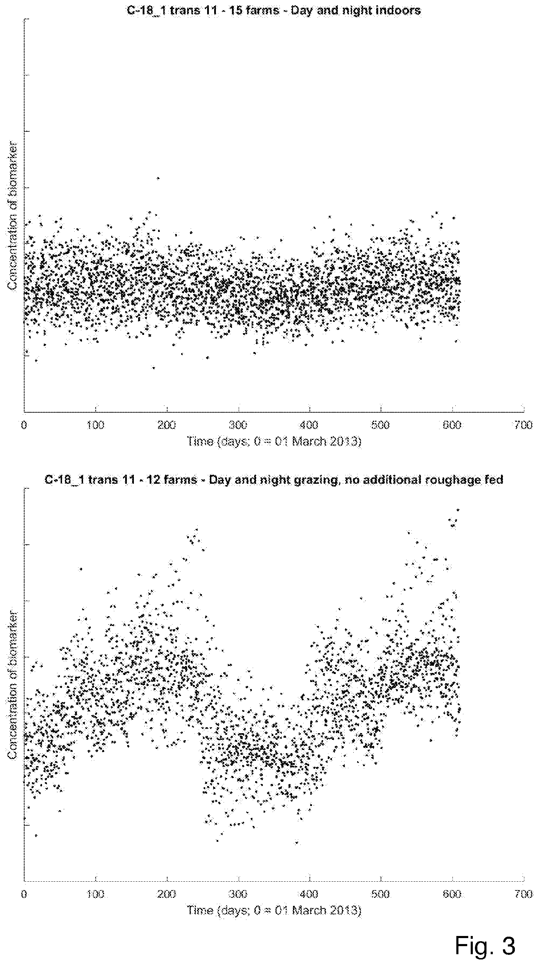

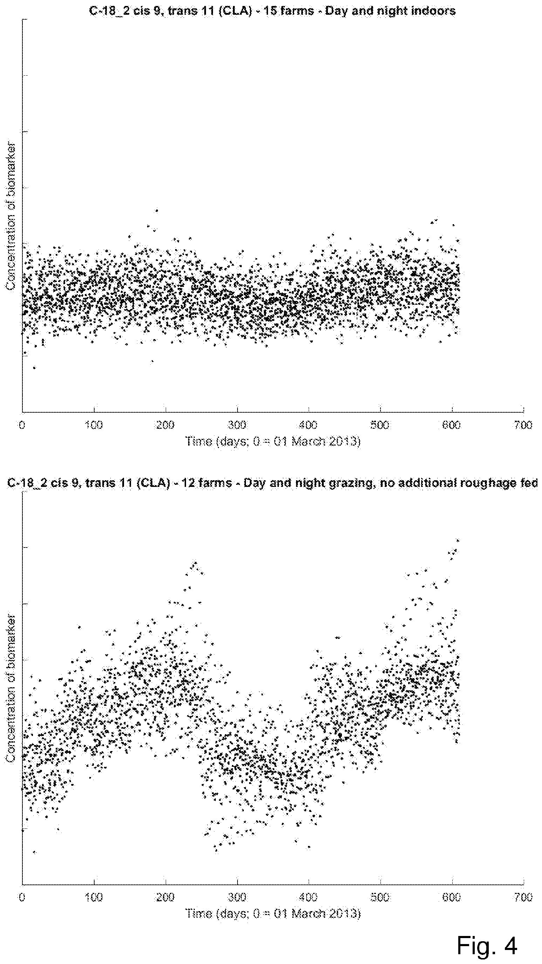

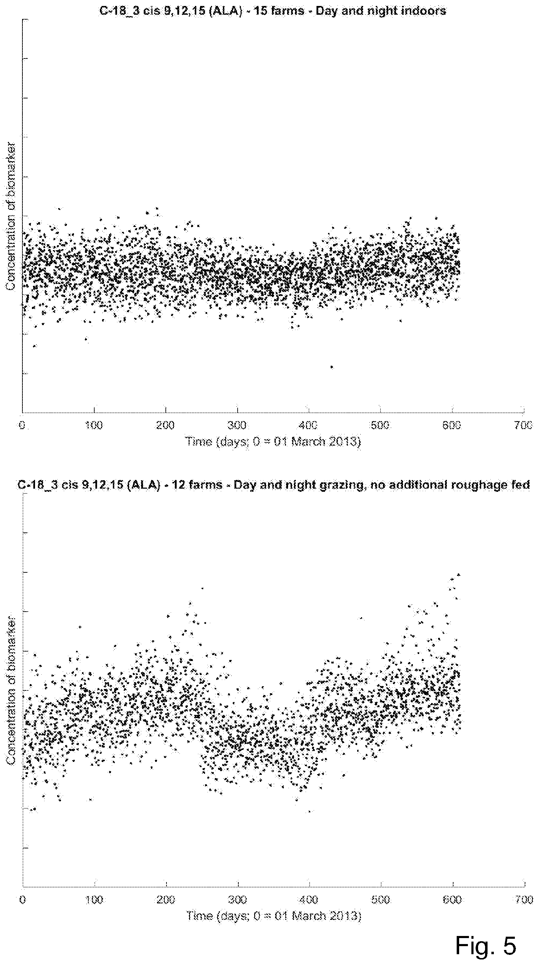

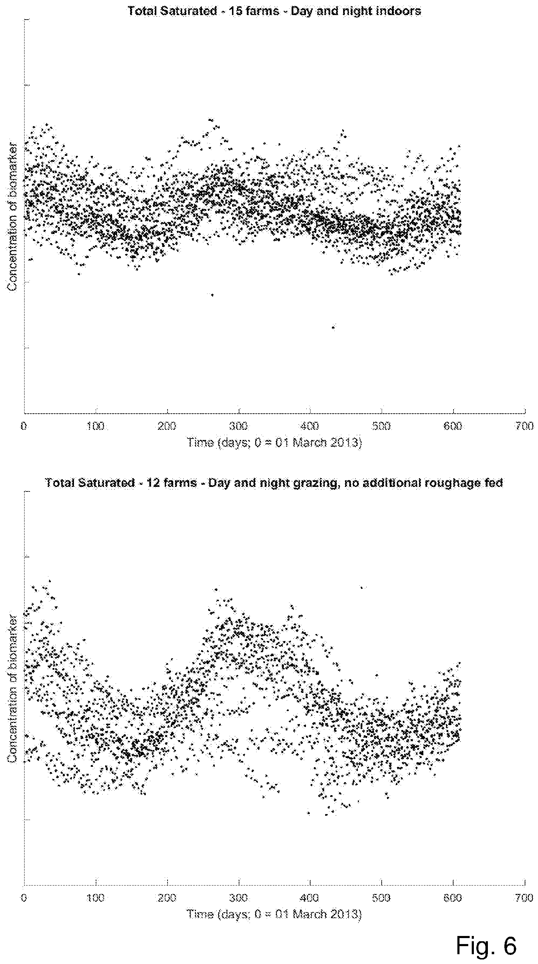

Figures 1-6 each show the concentration of biomarkers C14:0, C16:0, C18:1 trans 11, C18:2 cis 9, trans 11, C18:3 cis 9,12,15 and total saturated FAs respectively over time. The top of each figure represents the biomarker concentration of milk from 15 cow populations (farms) that are known to remain day and night indoors, while the bottom top of each figure represents the biomarker concentration of milk from 12 cow populations that are known to take in fresh grazed grass in the meadow for 24 hours a day without additional roughage over certain time periods.

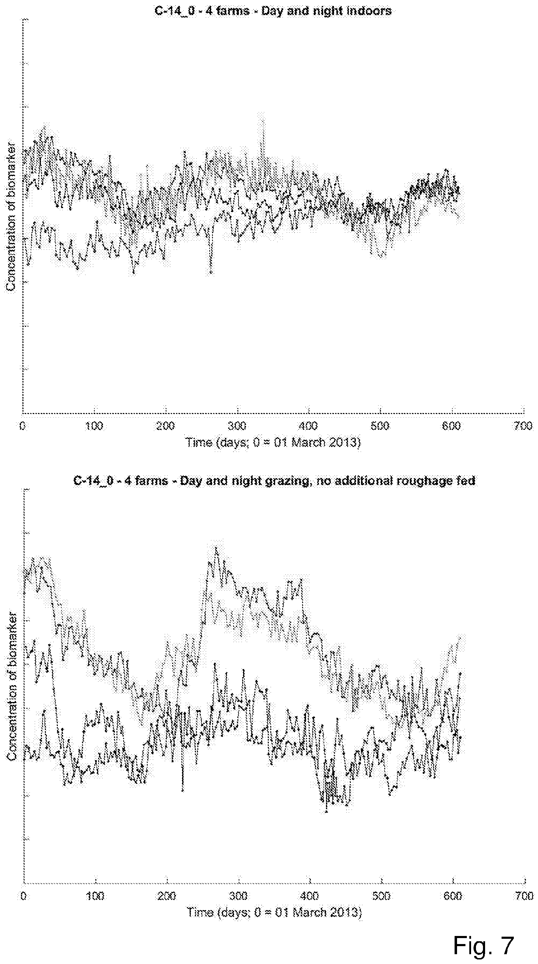

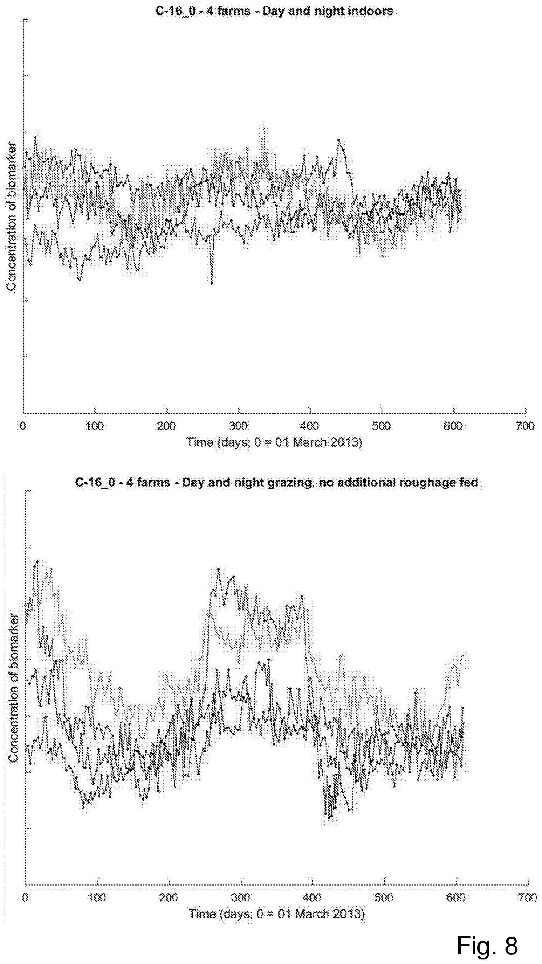

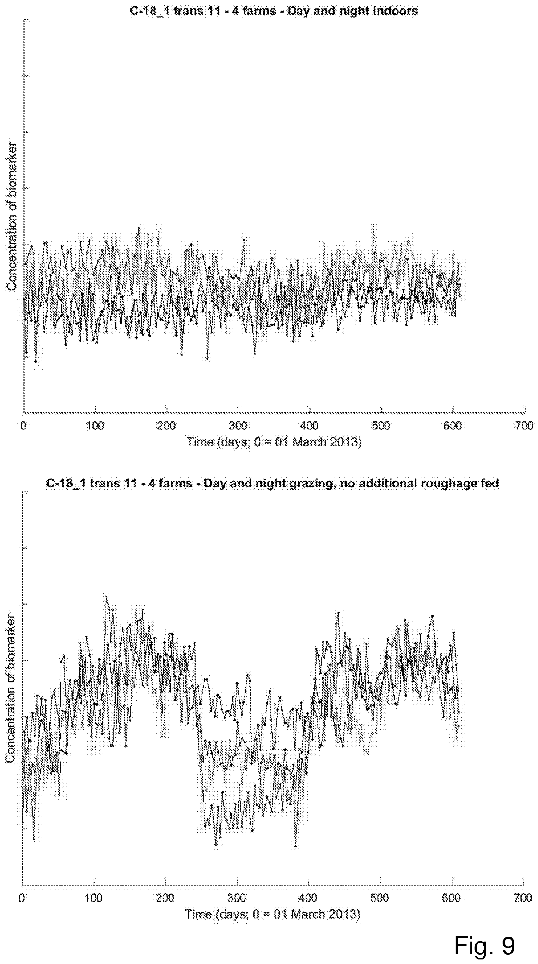

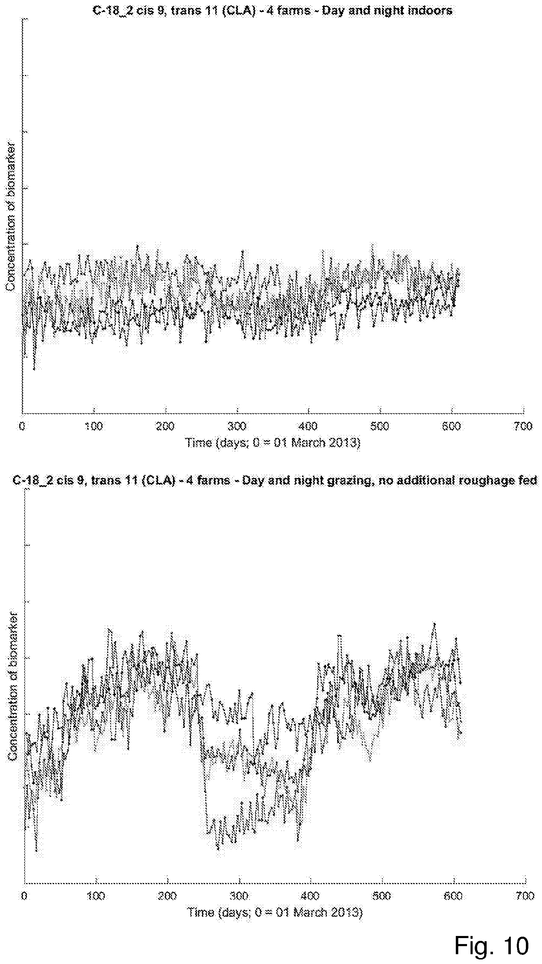

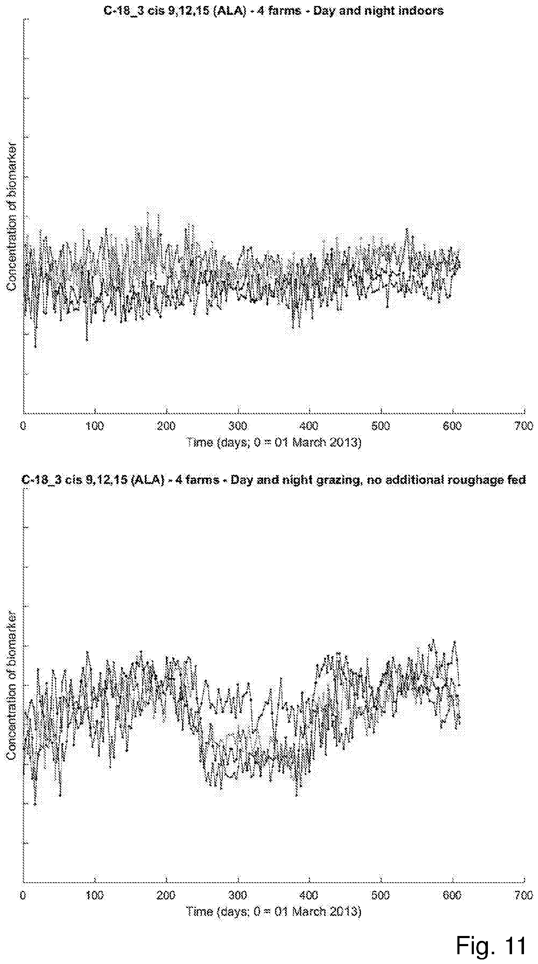

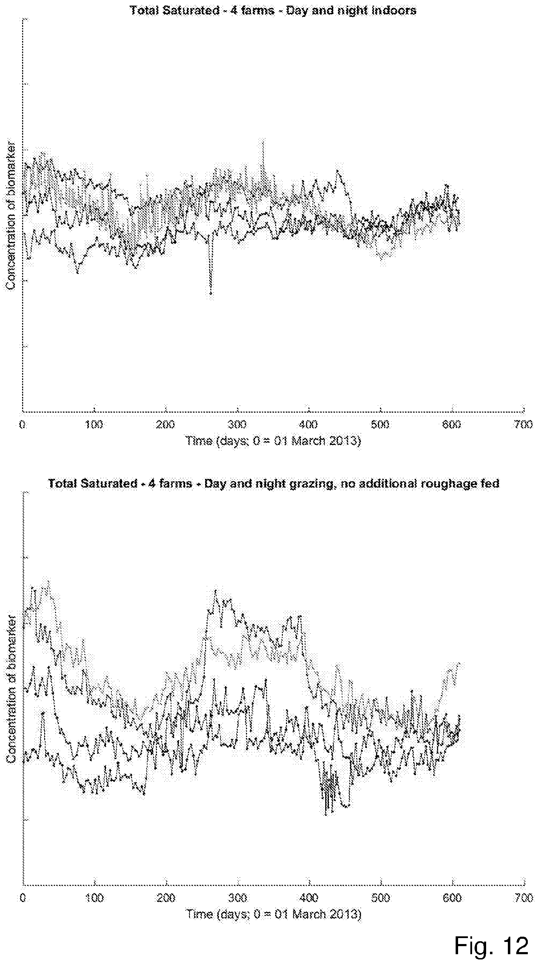

Figures 7-12 each show the concentration of biomarkers C14:0, C16:0, C18:1 trans 11, C18:2 cis 9, trans 11, C18:3 cis 9,12,15 and total saturated FAs respectively over time for four cow populations, wherein the data points originating from the same cow population are connected by a line. Figures like figures 1-12, may be used for the present invention.

By using the analytical methods known, other biomarkers may be found.

For a number of these specific biomarkers, the FT-IR analytical models are known in the art. For instance, models C14:0, C16:0 and total saturated FAs are available from Foss (Hillerød, Denmark) and are described in US 7,803,625 that includes also C18:1 trans 11 and C18:2 cis 9, trans 11 (CLA cis 9, trans 11) indirectly.

For these and other biomarkers such as C18:1 trans 11, C18:3 cis 9,12,15 and C18:2 cis 9, trans 11 FT-IR models can be determined using chemometrics known in the art.

As described herein-above, a number of samples for each time period are provided and analyzed. In addition, more than one biomarker may be used for the present invention. This results in data sets of the grazing time period per biomarker that may be compared to a data set of the reference time period per biomarker. Comparing these datasets results in several variables that could be combined with a statistical procedure, *e.g*. a (non)hierarchical clustering method such as principal component analysis (PCA). As such, step c) preferably comprises a (non)hierarchical clustering method such as PCA. PCA is a statistical method known in the field to group data sets. By using PCA in step c), components of the PCA, preferably the first principal component, may be used as the indicator for the fresh grazed grass intake.

In an embodiment of the present invention, the average amount C16:0 and the average amount of C18:1 trans 11 is calculated for a cow population over both the grazing (summer) time period and over the reference (winter) time period. PCA is performed on auto-scaled data of the following four variables: C16:0 summer average, C16:0 difference between winter and summer, C18:1 trans 11 summer average and C18:1 trans 11 difference between summer and winter. The score on the first principal component obtained from the PCA is applied as indicator of fresh grazed grass ingestion for the cow population over the grazing time period.

It was found that the color of the milk may also be used as a biomarker for the present invention. The color of milk obtained from meadow cows differs from milk obtained from cows that did not graze fresh grass.

The color of the milk can be determined by for instance determining the color index via analyzing the reflectance of the milk using a spectrocolorimeter (*e.g.* a Minolta CM 2002, Minolta France S. A., Carrières-sur-Seine, France), as described by F. Calderón et al. in J Dairy Sci. 2007 90(5):2335-46. The present inventors found that the color can also be predicted by using FT-IR models. These FT-IR models may also be developed using chemometrics known in the art.

The color of the milk as biomarker may be combined with the above-described biomarkers (*e.g*. the fatty acids). Thus, in a particular embodiment the present invention, the biomarker is one or more selected from the group of consisting of the fatty acids C14:0, C16:0, total saturated FAs, C18:1 trans 11, C18:2 cis 9, trans 11 and C18:3 cis 9,12,15 and of the color of the milk.

The present invention can improve the accuracy of the labeling of meadow milk by providing a sub-group of a group of farms that may still be labeled as providing meadow milk based on only one visit and visual inspection of the farms. As such, the sub-group of farms may be visited and inspected to re-evaluate the label without the requirement of more visits and visual inspections.

A particular aspect of the present invention is therefore a method for selecting a sub-group from a group of farms for additional (visual) inspection, said method comprising providing an indicator of fresh grazed grass ingestion for each farm in the group of farms followed by selecting a sub-group of the group of farms that have the lowest indicator of fresh grazed grass ingestion.

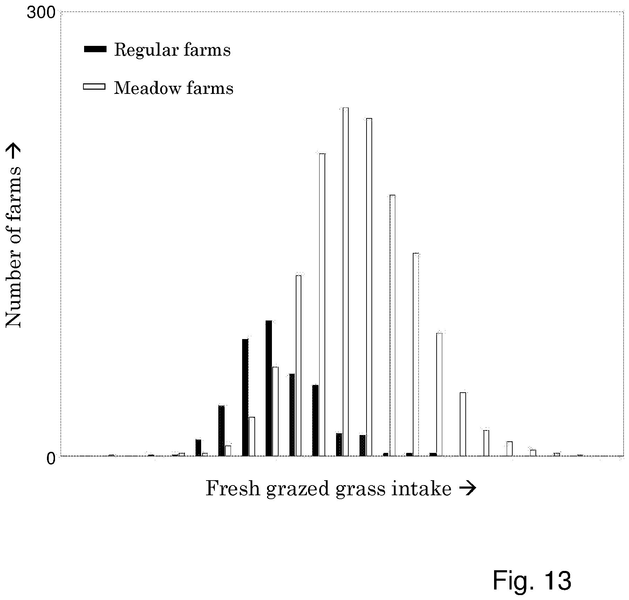

In figure 13, a plot is provided showing a distribution of the indicator obtained by the present invention for close to 2,000 farms located in the Netherlands. The farms are indexed according to their present label; *i.e.* meadow farms that have declared to have their cows in the pasture for at least six hours per day for a period of at least 120 days (white bars), regular farms that are known to keep the cows only or primarily inside (black bars). The Y-axis indicates the number of farms and the X-axis indicates the value for fresh grazed grass intake (left in figure low likelihood of fresh grazed grass intake, right in figure high likelihood of fresh grazed grass intake).

From a histogram like provided in figure 13, a sub-group of the farms can be selected for a more targeted visit and visual inspection. This prevents unnecessary visits and visual inspections that are likely performed when the visits are planned randomly. A higher accuracy in useful visits and visual inspections may reduce the overall amount of visits and visual inspections which in turn reduces overall costs and energy requirements for transportation.

In some cases, it may be preferred to confirm the fresh grazed grass ingestion or lack thereof for a cow population by visits and visual inspections. This may in particular be the case for cow populations that show a change over time in the indicator of fresh grazed grass ingestion.

In a particular aspect the present invention is therefore directed to providing indicators of fresh grazed grass ingestion for different time periods (*e.g*. for different years). As such, a change in fresh grazed grass ingestion of the cow population for a certain period may easily be detected without visiting and visually inspecting the cow population. The change in indicator according to the present invention has shown to be particularly accurate in estimating a risk of mislabeling of a cow population and/or the milk thereof.

A further aspect of the present invention is therefore a method of detecting a change in fresh grazed grass ingestion from a first grazing time period to a second grazing time period, comprising obtaining an indicator of fresh grazed grass ingestion for each time period, followed by comparing the indicator of fresh grazed grass ingestion over the first time period to the indicator of fresh grazed grass ingestion over the second time period to detect the change in fresh grazed grass ingestion.

## Claims

1. Method for determining fresh grazed grass intake over a grazing time period for a cow population, said method comprising the steps of:
a) providing a number of milk samples that are collected from the cow population over said grazing time period and over a reference time period without fresh grass grazing;
b) analyzing said milk samples and determine the amount of one or more biomarkers present in each of the milk samples;
c) comparing the amount of one or more biomarkers present in the milk during said grazing time period to the amount of one or more biomarkers present in the milk during the reference time period to obtain an indicator of fresh grazed grass intake for the cow population over the grazing time period.

2. Method according to claim 1, wherein the number of milk samples analyzed for each time period is at least 10, preferably at least 25.

3. Method according to any of the previous claims, wherein said grazing time period is a summer period and the reference time period is a winter period, preferably wherein said grazing time period spans at least 30 days selected from days within the period of April to September and wherein the reference time period spans at least 30 days selected from days within the period of December to February.

4. Method according to any of the previous claims, wherein the amount of each biomarker is different in the grazing time period compared to the amount of the respective biomarker in the reference time period.

5. Method according to any of the previous claims, wherein one or more biomarkers comprise one or more fatty acids, preferably selected from the group consisting of C 14:0, C16:0, total saturated FAs, C18:1 trans 11, C18:2 cis 9, trans 11 and C18:3 cis 9,12,15.

6. Method according to any of the previous claim, wherein the biomarker comprises the color of the milk.

7. Method according to any of the previous claims, wherein step b) of analyzing said milk samples comprises infrared spectroscopy.

8. Method according to any of the previous claims, wherein step c) comprises (non)hierarchical clustering method such as principal component analysis (PCA) and said indicator is a score on a (first principal) component of this analysis.

9. Method according to any of the previous claims, wherein the cow population is located on a single farm.

10. Method of detecting a change in fresh grazed grass intake from a first grazing time period to a second grazing time period, comprising providing an indicator of fresh grazed grass ingestion for each time period with a method according to any of the previous claims, followed by comparing the indicator of fresh grazed grass intake over the first time period to the indicator of fresh grazed grass intake over the second time period to detect the change in fresh grazed grass intake.

11. Method according to claim 10, wherein the change in fresh grazed grass intake from a first grazing time period to a second grazing time period is determined for each farm in a group of farms, followed by selecting a sub-group of the group of farms that have the highest change in fresh grass ingestion for visual inspection.

12. Method for selecting a sub-group from a group of farms for visual inspection, said method comprising providing an indicator of fresh grazed grass intake in accordance with any of claims 1-9 for each farm in the group of farms, followed by selecting a sub-group of the group of farms that have the lowest indicator of fresh grazed grass intake.

## Patentansprüche

1. Verfahren zum Bestimmen der Einnahme von frischem geweidetem Gras über einen Weidezeitraum für eine Kuhpopulation, das Verfahren umfassend die Schritte von:
a) Bereitstellen einer Anzahl von Milchproben, die von der Kuhpopulation über den Weidezeitraum und über einen Referenzzeitraum ohne frisches-Gras-Weiden gesammelt werden;
b) Analysieren der Milchproben und Bestimmen der Menge eines oder mehrerer Biomarker, die in jeder der Milchproben vorhanden sind;
c) Vergleichen der Menge eines oder mehrerer Biomarker, die in der Milch über den Weidezeitraum vorhanden sind, mit der Menge eines oder mehrerer Biomarker, die in der Milch über den Referenzzeitraum vorhanden sind, um einen Indikator für Einnahme von frischem geweideten Gras für die Kuhpopulation über den Weidezeitraum zu erhalten.

2. Verfahren nach Anspruch 1, wobei die Anzahl von Milchproben, analysiert für jeden Zeitraum, wenigstens 10, vorzugsweise wenigstens 25 ist.

3. Verfahren nach einem der vorhergehenden Ansprüche, wobei der Weidezeitraum ein Sommerzeitraum und der Referenzzeitraum ein Winterzeitraum ist, wobei vorzugsweise der Weidezeitraum sich über wenigstens 30 Tage, ausgewählt aus Tagen innerhalb des Zeitraumes von April bis September, erstreckt und wobei sich der Referenzzeitraum über wenigstens 30 Tage, ausgewählt aus Tagen innerhalb des Zeitraumes von Dezember bis Februar, erstreckt.

4. Verfahren nach einem der vorhergehenden Ansprüche, wobei die Menge jedes Biomarkers in dem Weidezeitraum verglichen mit der Menge des entsprechenden Biomarkers in dem Referenzzeitraum unterschiedlich ist.

5. Verfahren nach einem der vorhergehenden Ansprüche, wobei ein oder mehrere Biomarker eine oder mehrere Fettsäuren umfassen, vorzugsweise ausgewählt aus der Gruppe bestehend aus C14:0, C16:0, vollständig gesättigten FS, C18:1 trans 11, C18:2 cis 9, trans 11 und C18:3 cis 9,12,15.

6. Verfahren nach einem der vorhergehenden Ansprüche, wobei der Biomarker die Farbe der Milch umfasst.

7. Verfahren nach einem der vorhergehenden Ansprüche, wobei Schritt b) des Analysierens der Milchproben Infrarotspektroskopie umfasst.

8. Verfahren nach einem der vorhergehenden Ansprüche, wobei Schritt c) (nicht-)hierarchisches Clustering-Verfahren, so wie Hauptkomponentenanalyse (PCA) umfasst und der Indikator eine Punktzahl einer (ersten Haupt-) Komponente dieser Analyse ist.

9. Verfahren nach einem der vorhergehenden Ansprüche, wobei die Kuhpopulation auf einem einzelnen Bauernhof eingegrenzt ist.

10. Verfahren zum Nachweisen einer Änderung in der Einnahme von frischem geweidetem Gras von einem ersten Weidezeitraum zu einem zweitem Weidezeitraum, umfassend Bereitstellen eines Indikators für Aufnahme von frischem geweidetem Gras für jeden Zeitraum mit einem Verfahren nach einem der vorhergehenden Ansprüche, gefolgt vom Vergleichen des Indikators für Einnahme von frischem geweidetem Gras über den ersten Zeitraum gegenüber dem Indikator für Einnahme von frischem geweidetem Gras über den zweiten Zeitraum, um die Änderung in der Einnahme von frischem geweidetem Gras zu erfassen.

11. Verfahren nach Anspruch 10, wobei die Änderung in der Einnahme von frischem geweidetem Gras von einem ersten Weidezeitraum zu einem zweitem Weidezeitraum für jeden Bauernhof in einer Gruppe von Bauernhöfen bestimmt wird, gefolgt vom Auswählen einer Untergruppe der Gruppe von Bauernhöfen, die die höchste Änderung in der Aufnahme von frischem geweidetem Gras hat, zur Sichtprüfung.

12. Verfahren zum Auswählen einer Untergruppe aus einer Gruppe von Bauernhöfen zur Sichtprüfung, das Verfahren umfassend Bereitstellen eines Indikators für Einnahme von frischem geweidetem Gras in Übereinstimmung mit einem der Ansprüche 1 - 9 für jeden Bauernhof in der Gruppe von Bauernhöfen, gefolgt vom Auswählen einer Untergruppe aus der Gruppe der Bauernhöfe mit dem niedrigsten Indikator für die Einnahme von frischem geweidetem Gras.

## Revendications

1. Méthode de détermination de la consommation d'herbe pâturée fraîche sur une période de temps de pâturage d'une population de vaches, ladite méthode comprenant les étapes de :
a) fourniture d'un certain nombre d'échantillons de lait qui sont collectés à partir de la population de vaches sur ladite période de temps de pâturage et sur une période de temps de référence sans pâturage d'herbe fraîche ;
b) analyse desdits échantillons de lait et détermination de la quantité d'un ou de plusieurs biomarqueurs présents dans chacun des échantillons de lait ;
c) comparaison de la quantité du ou des biomarqueurs présents dans le lait pendant ladite période de temps de pâturage avec la quantité du ou des biomarqueurs présents dans le lait pendant la période de temps de référence pour obtenir un indicateur de la consommation d'herbe pâturée fraîche de la population de vaches sur la période de temps de pâturage.

2. Méthode selon la revendication 1, dans laquelle le nombre d'échantillons de lait analysés pour chaque période de temps est d'au moins 10, de préférence d'au moins 25.

3. Méthode selon l'une quelconque des revendications précédentes, dans laquelle la période de temps de pâturage est une période d'été et la période de temps de référence est une période d'hiver, de préférence dans laquelle ladite période de temps de pâturage s'étend sur au moins 30 jours choisis parmi les jours dans la période allant d'avril à septembre et dans laquelle la période de temps de référence s'étend sur au moins 30 jours choisis parmi les jours dans la période allant de décembre à février.

4. Méthode selon l'une quelconque des revendications précédentes, dans laquelle la quantité de chaque biomarqueur est différente pendant la période de temps de pâturage comparativement à la quantité des biomarqueurs respectifs pendant la période de temps de référence.

5. Méthode selon l'une quelconque des revendications précédentes, dans laquelle un ou plusieurs biomarqueurs comprennent un ou plusieurs acides gras, de préférence choisis dans le groupe constitué par C14:0, C16:0, FA saturés totaux, C18:1 trans 11, C18:2 cis 9, trans 11 et C18:3 cis 9,12,15.

6. Méthode selon l'une quelconque des revendications précédentes, dans laquelle le biomarqueur comprend la couleur du lait.

7. Méthode selon l'une quelconque des revendications précédentes, dans laquelle l'étape b) d'analyse desdits échantillons de lait comprend la spectroscopie infrarouge.

8. Méthode selon l'une quelconque des revendications précédentes, dans laquelle l'étape c) comprend une méthode de regroupement (non) hiérarchique telle qu'une analyse en composantes principales (ACP) et ledit indicateur est un score sur une (première) composante (principale) de cette analyse.

9. Méthode selon l'une quelconque des revendications précédentes, dans laquelle la population de vaches est localisée dans une seule ferme.

10. Méthode de détection d'une variation de consommation d'herbe pâturée fraîche entre une première période de temps de pâturage et une seconde période de temps de pâturage, comprenant
la fourniture d'un indicateur d'ingestion d'herbe pâturée fraîche pour chaque période de temps à l'aide d'une méthode selon l'une quelconque des revendications précédentes, suivie par
la comparaison de l'indicateur de consommation d'herbe pâturée fraîche sur la première période de temps avec l'indicateur de consommation d'herbe pâturée fraîche sur la seconde période de temps pour détecter la variation de consommation d'herbe pâturée fraîche.

11. Méthode selon la revendication 10, dans laquelle la variation de consommation d'herbe pâturée fraîche entre une première période de temps de pâturage et une seconde période de temps de pâturage est déterminée pour chaque ferme d'un groupe de fermes, suivie par la sélection d'un sous-groupe du groupe de fermes présentant la variation d'ingestion d'herbe pâturée fraîche la plus élevée à des fins d'inspection visuelle.

12. Méthode de sélection d'un sous-groupe dans un groupe de fermes à des fins d'inspection visuelle, ladite méthode comprenant la fourniture d'un indicateur d'ingestion d'herbe pâturée fraîche selon l'une quelconque des revendications 1 à 9 pour chaque ferme dans le groupe de fermes, suivie par la sélection d'un sous-groupe du groupe de fermes présentant l'indicateur de consommation d'herbe pâturée fraîche le plus bas.
